# EUROPEAN PATENT APPLICATION

(11) **EP 3 432 606 A1**
(43) Date of publication of application: **23.01.2019**
(21) Application number: 18161012.2
(22) Date of filing: 09.03.2018
(51) Int. Cl.: H04R 25/00, A61F 11/04, G01S 17/89, H04S 7/00

(54) **HEARING AID SYSTEM**

(71) Applicant: Oticon A/S, 2765 Smørum (DK)
(72) Inventor: Pontoppidan, Niels Henrik, DK-2765 Smørum (DK); Memic, Anida, DK-2765 Smørum (DK); Johansen, Benjamin, DK-2765 Smørum (DK); Wang, Yang, DK-2765 Smørum (DK); Thorén, Elisabet Sundewall, DK-2765 Smørum (DK); Petersen, Michael Kai, DK-2765 Smørum (DK); Petersen, Eline Borch, DK-2765 Smørum (DK); Griful, Sergi Rotger, DK-2765 Smørum (DK); Tietz, Lukas, DK-2765 Smørum (DK); Valdes, Alejandro Lopez, DK-2765 Smørum (DK); Hietkamp, Renskje K., DK-2765 Smørum (DK); Westergård, Bo, DK-2765 Smørum (DK); Laplante-Lévesque, Ariane, DK-2765 Smørum (DK); Cleveland Nielsen, Annette, DK-2765 Smørum (DK); Bramsløw, Lars, DK-2765 Smørum (DK); Meedom, Niels Henrik, DK-2765 Smørum (DK); Bhuiyan,Tanveer, DK-2765 Smørum (DK)
(74) Representative: William Demant

(57) **Abstract**

The invention concerns a hearing aid system that comprises a hearing device operationally connected to a visual signal output transducer. The hearing device comprises an audio signal input, for receiving an electric audio input signal, wherein the electric audio input signal has characteristics that depend on the sound environment and therefore represents information about the sound environment of the hearing device. The hearing device comprises an audio input signal processing unit operationally connected to the audio signal input. The audio input signal processing unit is configured to process the electric audio input signal, wherein processing the electric audio input signal includes
- analyzing the electric audio input signal with respect to the information about the sound environment represented by the characteristics of the electric audio input signal,
- extracting a signal fragment from the analyzed electric audio input signal, wherein characteristics of the extracted signal fragment represent information about the sound environment,
- generating a visual signal from the extracted signal fragment, wherein the visual signal depicts information about the sound environment represented by the extracted signal fragment, and
- providing a processed electric audio signal or the visual signal or both.

The hearing device comprises an audio signal output transducer that is operationally connected to the audio input signal processing unit. The audio signal output transducer is configured to provide an audio output signal that is based on the processed electric audio input signal and that can be perceived as sound by a user.

The hearing device comprises a visual signal output transducer that is operationally connected to the audio input signal processing unit. The visual signal output transducer is configured to provide a visual output signal that is based on the visual signal and that can be perceived as visual input by a user.

## Description

### TECHNICAL FIELD

The invention relates to a hearing aid system, a training arrangement and a method for providing a visual output signal.

### BACKGROUND

A hearing device is typically worn in or behind the ear of a hearing-impaired person. A hearing device is configured to receive sound from an ambient environment and to emit a processed sound signal by means of a speaker, sometimes also called receiver, into the ear canal of a hearing-impaired person, thus, stimulating the hearing-impaired person's eardrum. The processed sound signal may at least partly compensate for a user's hearing loss and, thus, the hearing experience of a hearing-impaired person may be significantly improved.

A hearing device typically comprises an input transducer, a signal processing unit and an output transducer. The input transducer can be a microphone and is configured to receive sound from an ambient environment and to convert the received acoustic signal into an electric audio input signal. The electric audio input signal typically is decomposed into a number of frequency bands and processed in the signal processing unit by executing processing algorithms that may result, e.g., in filtering, noise reduction or feedback cancellation in specific frequency bands. After processing the electric audio input signal, the signal processing unit provides an electric audio output signal which is fed to the output transducer. The output transducer, in turn, provides an output signal that is based on the electric audio output signal and that can be perceived as sound by a user of the hearing aid system.

A hearing device can be part of a hearing aid system that may further include another electronic device that is operationally connected to the hearing device. The hearing device and the other electronic device may interact/communicate with each other e.g. by exchanging data signals.

### SUMMARY

It is an object of the invention to provide a useful hearing aid system.

According to a first aspect of the invention, the object is achieved by a hearing aid system comprising a hearing device operationally connected to a visual signal output transducer. The hearing device comprises an audio signal input, for receiving an electric audio input signal, wherein the electric audio input signal has characteristics that depend on the sound environment and therefore represents information about the sound environment of the hearing device. The sound signal that is represented by characteristics of the received electric audio input signal, is typically a superposition of sound signals emitted by sound sources of the surrounding sound environment.

The hearing device comprises an audio input signal processing unit operationally connected to the audio signal input. The audio input signal processing unit is configured to process the electric audio input signal, wherein processing the electric audio input signal includes
- analyzing the electric audio input signal with respect to the information about the sound environment represented by the characteristics of the electric audio input signal,
- extracting a signal fragment from the analyzed electric audio input signal, wherein characteristics of the extracted signal fragment represent information about the sound environment,
- generating a visual signal from the extracted signal fragment, wherein the visual signal depicts information about the sound environment represented by the extracted signal fragment, and
- providing a processed electric audio signal or the visual signal or both.

The hearing device comprises an audio signal output transducer that is operationally connected to the audio input signal processing unit. The audio signal output transducer is configured to provide an audio output signal that is based on the processed electric audio input signal and that can be perceived as sound by a user.

The hearing device comprises a visual signal output transducer that is operationally connected to the audio input signal processing unit. The visual signal output transducer is configured to provide a visual output signal that is based on the visual signal and that can be perceived as visual input by a user.

Thus, according to the invention, information about the sound environment that is represented by the characteristics of a received electric audio input signal is transformed into a visual signal that depicts the information about the sound environment contained in the received electric audio input signal. In other words, the hearing aid system converts information about the sound environment that are contained in a received electric audio input signal into a visual signal that depicts the information about the sound environment represented by the received electric audio input signal.

Accordingly, missing information can be provided as visual input to a user of the hearing aid system through the visual output transducer if the user of the hearing device has difficulties, e.g., in understanding speech, recognizing objects or localizing objects or object types . Hence, information lost due to hearing impairment can be visually presented to a user. The hearing aid system might also help a user to localize and to identify sound source and, thus, may also improve the self-confidence of a user of a hearing device in everyday life.

Preferably, the audio signal input is connected to an input transducer that is configured to receive an incoming sound signal and to convert the received sound signal into an electric audio input signal. Thus, the electric audio input signal is a sound-representing electric audio signal. The input transducer can for example be a microphone that can also be a directional microphone with two microphones or two sound inlets. Directional hearing devices analyze incoming sound signals received at the two microphones or the two sound inlets. Directional processing can also be achieved by using the electronic output of two separate omnidirectional microphones of a binaural hearing device that comprises a left and a right hearing device. By analyzing phase, amplitude or sound pressure gradient of two received sound signals, information on directionality can be retrieved. In particular, characteristic directional patterns and their adjustment during use of a hearing device can be used to adapt the hearing device directionality to different listening situations. Alternatively, the audio signal input can be connected to a signal receiver for wireless reception of electric audio signals, e.g., via Bluetooth.

The audio input signal processing unit receives the electric audio input signal from the audio signal input and is configured to process the electric audio input signal. Processing of the electric audio input signal includes analyzing the electric audio input signal. In particular, the electric audio input signal is analyzed with respect to the information about the sound environment represented by the characteristics of the electric audio input signal. Characteristics of the electric audio input signal comprise signal frequency, signal amplitude, signal phase and signal noise level. Different sound sources emit characteristic sound signals.

When a sound signal is received by an input transducer and converted into a respective electric audio input signal, the characteristics of the electric audio input signal represents information about the received sound signal.

An analysis of the electric audio input signal can be performed by using signal processing techniques such as frequency filtering or noise reduction. An analysis of the electric audio input signal can also be performed by executing a pattern recognition algorithm. For example, a speech pattern could be analyzed. Also the sound of a car or of a singing bird might lead to characteristic patterns in the electric audio input signal. Moreover, an analysis of the electric audio input signal can be used to differentiate between signal noise and meaningful sound information represented by the electric audio input signal.

Processing of the electric audio input signal includes extracting a signal fragment of the analyzed electric audio input signal. The extracted signal fragment is identified with specific information about the sound environment, e.g., information about a specific sound source. Hence, a signal fragment is a limited signal sequence or section of the received electric audio input signal having a start point and an end point, preferably in time or frequency. The start point of and the end point of a signal fragment can be the start point and end point of a sound signal emitted from a specific sound source that contributed to the sound environment of the hearing device. Typically such a signal fragment spans over a variable time duration according to the time duration of the sound signal emitted by the specific sound source.

Preferably, the signal fragment of the analyzed electric audio input signal represents meaningful sound information. In particular, a signal fragment of the analyzed electric audio input signal represents sound information that can be identified with a specific sound source that contributed to the superposition of sound signals emitted by sound sources of the surrounding sound environment. For example, the signal fragment of the analyzed electric audio input signal may be identified as representing a specific word spoken by the user of the hearing device or a respective communication partner. The signal fragment of the analyzed electric audio input signal may also be identified as an object like a car or as a singing bird. It may be possible to apply a pattern recognition algorithm to analyze patterns and match the analyzed patterns to different words or objects.

Processing of the electric audio input signal includes generating a visual signal from the extracted signal fragment. The visual signal depicts the information about the sound environment represented by the extracted signal fragment. In other words, the visual signal depicts information about the sound environment that are represented by the signal fragment of the analyzed electric audio input signal from which the visual signal has been generated from. The visual signal can be an analog or a digital video signal or it can encode a graphics or image or it can encode text information. The visual signal can also encode a graphical object like a dot, an arrow or a square. The visual signal can also encode a voice command.

Preferably, processing the electric audio input signal also includes executing one or more processing algorithms to obtain a processed electric audio input signal that, e.g., is filtered compressed, shifted or amplified in selected frequency bands. After processing the processed electric audio signal is provided, e.g., at an output of the audio input signal processing unit for further transmission.

Processing of the electric audio input signal includes providing a processed electric audio signal or the visual signal. Processing of the electric audio input signal also includes providing both a processed electric audio signal and the visual signal. The latter case is of particular interest if the processed electric audio signal and the visual signal are correlated. For example, if the visual signal depicts information about the sound environment represented by the extracted signal fragment, the visual signal can be complemented with a corresponding processed electric audio signal.

Based on the processed electric audio signal, the audio signal output transducer provides an audio output signal that can be perceived as sound by a user. The output transducer can be a receiver that emits sound to stimulate a user's eardrum. A respective hearing device can be implemented e.g. as a behind-the-ear (BTE) hearing device which typically has the microphone arranged behind the ear of a user or as an in-the-ear (ITE) hearing device which typically has the microphone arranged in the ear of a user. In both cases, a speaker is typically placed inside a user's ear canal to stimulate the eardrum.

The hearing device can be a single hearing device either worn on the left or the right ear of a user. The hearing device can also be a binaural hearing device comprising two hearing devices one of the worn on the left ear and the other on the right ear of a user. The left and right hearing device typically exchange data signals to adapt their performance more accurately to specific listening situations. With binaural hearing devices selective listening can more easily be achieved, speech intelligibility can be improved in difficult listening situations and the ability to tell the direction of sound can be improved with respect to a monoaural hearing device.

The hearing device can also comprise an implantable part such as in case of a cochlea implant or an auditory brainstem implant. In a cochlea implant, the output transducer can comprise a stimulation pulse generator and an electrode array comprising a number of electrodes for stimulating the cochlea nerve with electric pulses. If the hearing aid system is an auditory brainstem implant, the output transducer is configured to use electric stimulation to stimulate the brainstem of a user. In all cases, the audio output signal provided by the output transducer can be perceived as sound by a user.

The visual signal output transducer is configured to provide a visual output signal that is based on the visual signal and that can be perceived as visual input by a user. In particular, the visual output signal is provided during hearing aid system operation while the hearing device is mounted on a user. The visual output signal can represent visual input in form of text, e.g., a text deduced from spoken words represented by the received electric audio input signal. The visual output signal can also represent visual input in form of a video, an animation, a graphics or an image. For example, if the signal fragment extracted from the analyzed electric audio input signal is identified with car noise, a visual output signal representing a car can be provided as visual input to a user.

The electric audio input signal may also represent a voice command. The electric audio input signal can be analyzed by the audio input signal processing unit and respective signal fragments can be extracted. The visual signal generated from the extracted signal fragments can encode the execution information of the voice command represented by the received electric audio input signal. The visual signal output transducer converts the encoded execution information into a respective visual output signal which is then provided to a user as visual input. For example, a voice command can concern cleaning of the hearing device. A respective visual output signal can represent a video showing how to clean the hearing device, wherein the video is presented as visual input, e.g., to the user.

The visual signal output transducer can be an external electric device that can be wire-connected or wireless-connected to the hearing device for allowing an exchange of data signals between both devices.

The visual signal output transducer can provide the visual output signal through a display such that a user perceives the visual input through the display. The visual signal output transducer can also be implanted into a user's body, e.g., as a stimulation unit comprising stimulation electrodes for stimulating the user's brain such that the user perceives the information about the sound environment as visual input. The visual signal output transducer can also be a hardware part of the hearing device.

In a preferred embodiment, the hearing aid device of the hearing aid system is a binaural hearing device comprising a left hearing device and a right hearing, wherein the left hearing device and right hearing device are operationally connected to each other and to the visual signal output transducer.

In a preferred embodiment, the hearing aid system further comprises
- a visual signal input, for receiving a visual input signal, and
- a visual input signal processing unit operationally connected to the visual signal input and to the electric audio input signal processing unit and to the visual signal output transducer, for
- processing the visual input signal, wherein processing the visual input signal includes modifying the visual input signal in dependence on the visual signal provided by the electric audio input signal processing unit, and
- providing a modified visual input signal to the visual signal output transducer.

The visual signal input can be, e.g., a camera, LIDAR (light detection and ranging) or an auxiliary device for receiving a visual input signal. The visual input signal can be processed in the visual input signal processing unit. In particular, the visual signal is used to modify the visual input signal. For example, the visual signal may encode an arrow which can indicate a location of an object in the visual output signal. A user who perceives the visual input according to the modified visual output signal, perceives the visual input according to the visual input signal that has been modified with a respective visual signal. In particular, if a visual output signal that is based on a modified visual output signal and a complementary audio output signal are provided to a user, the visual input signal can be modified in that the visual output signal is synchronized with respect to the audio output signal.

Optionally, the audio input signal processing unit may comprises at least one of an
- audio input speech identifier, for identifying speech information represented by a signal fragment extracted from the analyzed electric audio input signal and for transforming the signal fragment that represents speech information into a text representing output signal,
- auditory object identifier, for identifying the sound information about the sound environment represented by a signal fragment extracted from the analyzed electric audio input signal with an auditory object and transforming the signal fragment that represents the auditory object information into an auditory object representing output signal, and
- auditory object localizer, for extracting position data from the analyzed electric audio input signal, wherein the position data represent a position relative to the hearing device of an auditory object represented by a respective signal fragment.

The audio input speech identifier provides a text representing signal, i.e. a signal that represents text. The text representing signal represents the speech information represented by the electric audio input signal. For obtaining a text representing signal, speech information represented by a signal fragment extracted from the analyzed electric audio input signal is identified and transformed into a respective text representing signal. The speech information represented by a signal fragment can originate from words, e.g., spoken by a user of the hearing aid system or by a communication partner.

The auditory object identifier is configured to identify sound information represented by a signal fragment extracted from the analyzed electric audio input signal with a sound object. A sound object can be an object that can be identified as the sound source. For example the noise of a car can be identified with the car itself. The singing of a bird can be identified with the bird who sings.

The auditory object localizer is configured to extract position data from characteristics of the analyzed electric audio input signal. Position data may comprise spatial coordinates relative to the hearing device which define the position of an auditory object with respect to the hearing device. In other words, the auditory object localizer associates an auditory object with a position relative to hearing device. Preferably, the position data provided for further processing. In particular, position data may represent data that represent the direction of impeding sound relative to the hearing aid system. The auditory object localizer may also access directionality characteristics retrieved by a directional microphone of a hearing device or by a binaural hearing device comprising a left and a right hearing device.

Optionally, the visual input signal processing unit may comprise at least one of a
- visual input signal speech analyzer, for analyzing the visual input signal with respect to lip movements and for providing a lip movement representing output signal,
- visual object analyzer, for analyzing the visual input signal with respect to a visual object and for providing a visual object representing output signal, and
- visual object localizer, for extracting spatial coordinates from the visual input signal that define a position of a visual object represented by the visual input signal with respect to the position of the hearing device.

The visual input signal speech recognizer is configured to identify lip movements represented by the visual input signal. The lip movements can be analyzed by setting artificial markers on the lips represented by the visual input signal. The movement of the marker, and, thus, of the lips can be recorded to recognize the lip movements. The result of the lip movement analysis can be represented by a generated lip movement representing output signal. It is preferred, that the generated lip movement representing output signal is used to perform lip-to-text processing and that the output of the lip-to-text processing is fed to a text-to-speech synthesizer that is configured to generate a speech signal representing the lip-movements.

The visual object analyzer is configured to analyze the visual input signal with respect to visual objects represented by the visual input signal. Based on a recognized visual object represented by the visual input signal, a visual object representing output signal can be generated which can be used for further processing.

The visual object localizer is configured to extract spatial coordinates from the visual input signal that define a position or a direction of a visual object represented by the visual input signal with respect to the position of the hearing device. In other words, the visual object localizer is configured to associate a visual object represented by a visual input signal with a position relative to the hearing device.

Optionally, the visual signal output transducer comprises at least one of
- an artificial object positioner, that is configured to modify a visual input signal with a respective visual signal which represents information on positioning an artificial object in the visual output signal and to provide a modified visual output signal,
- a lip-movement modifier, that is configured to modify the lip movements represented in a visual input signal in dependence on a visual signal which represents speech information and to provide a modified visual output signal, and
- an object placement informer, that is configured to modify the visual input signal with an object placement informer signal that represents information about a placement of an object and to provide a modified visual output signal.

In general, the artificial object positioner, the lip-movement modifier and the object placement informer modify a visual input signal that represents an unaltered visual input with a visual signal. Hence, the modified visual output signal in general is a superposition of a real image or image sequence with a virtual image or image sequence. In particular, as the visual signal used for modifying is based on the information represented by characteristics of a signal fragment extracted from the electric audio input signal, the visual input signal is modified with respect to the information represented by the signal fragment extracted from the electric audio input signal.

The artificial object positioner modifies a visual input signal by means of a visual signal which represents information on where to position an artificial object, like a dot, a square, or an arrow, in the visual output signal. A respective visual signal can be an object representing signal provided by an auditory object identifier or position data representing a position or direction relative to the hearing device of an identified auditory object provided by an auditory object localizer. Based on an object representing signal and respective position data the visual input signal can be modified to signal to a user via, e.g., an arrow, that a certain type of auditory object has been identified at a certain position or a certain direction relative to the hearing device. It might be useful, to implement a colour coding scheme that relates a certain type of auditory object with a respective colour. For example, if a musical object is identified as well as its location, a dot may indicate the direction and a certain colour of the dot may indicate that it is a musical object. A possible colour-coding scheme may be implemented as blue for music, green for birds, red for cars or other motor vehicles, purple for voices and grey for noise. Eventually, the artificial object positioner provides a modified visual output signal.

The lip-movement modifier modifies the lip movements represented in a visual input signal in dependence on a visual signal which represents speech information. In particular, the lip movements represented in a visual input signal can be modified by a text representing signal provided by an audio input speech identifier. By operationally connecting the audio input speech identifier to a lip-movement modifier the text representing signal can be used to enhance the lip movements represented in a visual input signal and to provide a modified visual output signal. Thereby, visual speech cues can be more salient in the modified output signal that is provided as a visual output signal to a user. It is preferred, that the lip movements represented by the visual input signal are modified in real-time with respect to provided perceived visual output signal. It is of a particular advantage, if the lip movements are modified synchronous to a possible speech delay such that a user does not perceive a delay between the perceived audio output signal and the perceived visual input.

The object placement informer modifies the visual input signal with an object placement informer signal that represents information about a placement of an object. Via the audio signal input, an electric audio input signal representing sound information of an auditory object may be received. An auditory object identifier may provide a respective auditory object representing signal and an auditory object localizer may provide position data representing a position of a respective auditory object relative to the hearing device. Based on an auditory object representing signal and respective position data, an object placement informer signal may modify a visual input signal in that a user is alerted about an identified object, e.g., by an arrow included in the visual output signal pointing in the direction of the identified object.

It is preferred that the visual output signal is provided by means of video overlay, e.g., if the visual signal output transducer is implemented within glasses, a contact lens, a virtual reality headset, a portable electronic device in particular a Smartphone.

If the visual signal output transducer is implemented as a retinal implant or a post-retinal implant, the visual output signal or the modified visual output signal can be provided via direct brain stimulation. In some embodiments it may be useful to provide the visual output signal or the modified visual output signal via indirect brain stimulation, in particular deep brain stimulation.

Optionally, the hearing aid system comprises an eye movement tracker. Via eye tracking, the point of gaze or the motion of an eye relative to the head can be measured and analyzed. If the visual signal output transducer is a contact lens, the eye movement tracker can be implemented within the respective contact lens, too, to implement eye-attached tracking. If the visual signal output transducer is implemented within, e.g., glasses or a virtual reality headset, optical tracking can be implemented. In case of optical eye tracking, an infrared light of an infrared light source is reflected from the eye and sensed by, e.g., a video camera. The recorded information is then analyzed to retrieve the eye movement.

In an embodiment, the hearing aid system comprises a monitoring unit operationally connected to the visual signal output transducer or operationally connected to the audio signal output transducer or both, wherein the monitoring unit is configured to
- monitor a feature of the hearing aid system or of the received electric audio input signal or both,
- identify a potential need of assistance for a user of the hearing aid system based on the monitored feature of the hearing aid system or of the electric audio input signal or both,
- select an assistance output signal that represents assistance information related to the identified potential need of assistance from a set of assistance output signals and to provide the selected assistance output signal, wherein
the visual signal output transducer is configured to provide a visual output signal that is based on the selected assistance output signal or the audio signal output transducer is configured to provide an audio output signal that is based on the selected assistance output signal or both.

The monitoring unit can be integrated into the hearing device. It provides assistance information about when and how to maintain a respective hearing device. In particular, the monitoring unit provides an assistance output signal based on which instant assistance of how to solve or carry out a specific task is provided. It is preferred that a potential need of assistance can originate either from monitoring a feature of the hearing aid system or by voice prompts or the recognition of voice commands. Based on the identified potential need of assistance specific hearing device handling or maintenance tasks can be carried out when a potential need or problem occurs or when it is expected to occur.

Assistance can be provided audibly in form of the audio output signal that is based on a respective assistance output signal. A respective assistance can be a voice prompt received by a user of the hearing aid system through a receiver that is inserted in the user's ear canal.

Assistance can also be provided visually, e.g., through a visual signal output transducer that, e.g., has a display for presenting visual input represented by a visual output signal that is based on a selected assistance output signal. A respective visual signal output transducer can be a Smartphone or a virtual reality headset operationally connected to the hearing device. For example, an interactive map of the hearing device can be displayed on an electronic device, e.g., a Smartphone or tablet that is equipped with a camera. Image algorithms can be implemented to recognise hearing device components critical for the identified potential need of assistance and for the assistance information of what steps have to be performed in order to satisfy the identified potential need of assistance. In particular, a step-by-step guidance can be provided on a display, presenting tasks to be performed to satisfy an identified potential need of assistance. In particular step-by-step guidance can be provided for predefined scenarios. If, e.g., the visual signal output transducer is a Smartphone or another electronic device that has internet access, it can be useful to search the internet for content that may be of relevance for satisfying an identified potential need of assistance and to provide a visual output signal representing the found content as visual input to a user of the hearing device.

In particular, assistance may be provided visually and audibly to a user, e.g., in form of a video complemented with spoken explanations.

Features of the hearing aid system that may be monitored by the monitoring unit comprise the signal quality of the received signal or of the output signal, e.g., concerning the signal-to-noise ratio, electronic failure, software failure, the battery status, the fitting of the hearing device, the currently applied hearing device program or receiver occlusion. From the listed examples of monitored features, a potential need of assistance may be deduced. A potential need of assistance may also be identified by monitoring the received electric audio input signal. If a user or a communication partner asks for support, a potential need of assistance may be identified via voice prompt recognition. If for example, a user asks for help for, e.g., changing the battery, a respective assistance output signal can be selected according to the identified need.

The monitoring unit may comprise or access a memory comprising a predefined set of assistance output signals from which a respective assistance output signal can be selected according to an identified need of assistance.

A visual output signal that is based on the selected assistance output signal can be presented in form of a video or a graphic that explains how to solve or carry out a specific task to satisfy the potential need of assistance. An audio output signal that is based on the selected assistance output signal can be presented in form of a spoken explanation of how to solve a specific task to satisfy the potential need of assistance.

Optional, the monitoring unit comprises or is connected to a self-verification unit, for monitoring a feature of the hearing aid system according to pre-defined features. For example, if the user of the hearing device is a new hearing device user, the hearing device can comprise a program for setting up the hearing device. Via voice prompts and video a user can be informed about, e.g., when a specific task has to be carried out for the first time, like changing the battery or prompting a manual switch of a hearing device program in a specific listening situation.

Optional, the monitoring unit comprises or is connected to a fault detection unit, for automatic fault detection. Automatic fault detection can be performed continuously during hearing device operation. An assistance output signal generated by the fault detection unit may represent assistance information, e.g., related to that the receiver is filled with wax or that the hearing device is now properly mounted on a user. A respective assistance output signal may represent information on the steps to be performed to satisfy an automatically identified need of assistance. Thus, via a fault detection unit it is possible to provide self-verification or integrity checks for fault detection.

Optional, the monitoring unit comprises or is connected to a voice command recognition unit, to detect a potential need of assistance represented by the received electric audio input signal. The received electric audio input signal may represent a voice command articulated by a user of the hearing aid system or by another person. For example, a communication partner may help a user of the hearing device to mount a hearing device but cannot remember if a hearing device has to be mounted on the left or the right ear. A user may also ask for a battery status or how to change the battery of the hearing device.

Optional, the monitoring unit comprises or is connected to an assistance output signal selector that is configured to select an assistance output signal from a set of assistance output signals stored, e.g., in a memory that is optionally comprised in the monitoring unit. In particular, the assistance output signal selector is configured to select an assistance output signal from a set of assistance output signals based on an identified potential need of assistance.

In an embodiment, the monitoring unit is configured to identify a fulfilment of an identified potential need of assistance based on the monitored feature or the received electric audio input signal or both and to provide a need fulfilment output signal.

The identification of a fulfilment of an identified potential need of assistance based on the monitored feature can be performed automatically. For example, via a global positioning system (GPS) and a feedback analysis unit embedded in the hearing device it can be verified whether a hearing device is mounted properly and a respective need fulfilment output signal can be provided accordingly. By checking the battery status it can be automatically checked, whether the identified need of changing the battery has been satisfied.

The identification of a fulfilment of an identified potential need of assistance can also be based on the monitored electric audio input signal. The electric audio input signal can represent a user command indicating that an identified potential need of assistance has been satisfied. In other words, a user can verbally report that whether or not an identified potential need of assistance has been satisfied.

The audio output signal transducer can provide an audio output signal that is based on the need fulfilment output signal. For example, a respective audio output signal can represent information indicating that a task has been correctly performed. The visual output signal transducer can provide a visual output signal that is based on the need fulfilment output signal, too. For example, a respective visual output signal can represent information indicating that a task has been correctly performed.

In an embodiment, the monitoring unit is configured to record monitoring data representing at least one of an identified potential need of assistance, steps performed to satisfy an identified potential need of assistance and a fulfilment of an identified potential need of assistance.

The recorded monitoring data can be stored in an optionally comprised memory or send or transmitted via a wire or wireless connection to an external electronic device, e.g. via Bluetooth. The stored data can be accessed, e.g., by a hearing care professional for later analysis or by a manufacturer to improve features, fault detection mechanisms or quality mentoring of hearing devices. A hearing care professional can also collect the monitoring data directly via a wire or wireless link. The monitoring data may help a haring care professional to provide individualised follow-up care for a hearing device user and track a potential need for further intervention. In particular, by recording monitoring data it is possible to log internal errors identified by a fault detection unit that can be accessed by a hearing care professional.

A hearing aid system according to at least one of the aforementioned embodiments may also be used within a training arrangement. A respective training arrangement comprises
- a hearing aid system according to at least one of the aforementioned embodiments,
- a sound source for providing at least one directional sound signal,
- a user interface that is configured to record user action data representing a user action, and
- an evaluator that is operationally connected to at least one of the
   - sound source, to control the directional sound signal provided by the sound source,
   - user interface, to receive and evaluate the recorded user action data and to provide evaluation data representing an evaluation result, and
   - visual signal output transducer, that is configured to provide a visual output signal representing visual content that complements the directional sound signal or that is configured to provide a visual output signal based on the provided evaluation data or both to a user of the hearing aid system.

The sound source can be surround sound headphones or a speaker or speaker arrangement for providing a directional sound signal that can be perceived by a user of the training arrangement.

The user interface can comprise or can be connected to an eye movement tracker. Via eye tracking, the point of gaze or the motion of an eye relative to the head can be measured and analyzed. For example, if the visual signal output transducer is implemented within a virtual reality headset comprising or connected to an eye movement tracker, it can be tracked whether or not a user looks at an object that emits a sound signal. Thus, via an eye movement tracker it can be verified that a user found a correct sound source.

The user interface can comprise or can be connected to a controller. By pressing a button of the controller a correct sound source or object identification can be registered. For example, if a user looks at an identified sound source as detected by an eye movement tracker and presses a button of a controller it can be verified that a user found the correct sound source.

It is also possible that head tracking is implemented within a virtual reality headset. Head tracking can be implemented by utilizing accelerometers and gyroscopes integrated in a virtual reality headset. If via head tracking it is measured that a user is looking at a correct location and then presses a button of a controller, it can be verified that the user found the correct sound source.

In the described implementations of a user interface, user action data representing a user action are recorded. User action data can represent a tracked eye movement, a button press or the looking direction measured via head tracking.

Accordingly, the training arrangement can be used to train localizing or identifying sound sources. The training arrangement can also be used for users to learn how to position themselves with respect to different sound environment situations. The training arrangement can also be used for training communications strategies with respect to different communication partners or sound environments. The training arrangement can also be used for hearing rehabilitation training.

The evaluator can be a home video game console. The evaluator can also be a personal computer or an alternative suitable electronic device. Via the evaluator the sound source can be controlled, e.g., the directionality of a sound signal. In particular, different sound scenes such as a nature scene, a city scene, a home scene, and a restaurant scene can be provided to the sound source and a respective sound signal can be emitted. A sound scene can be rather complex, e.g., comprising simultaneous sound sources and directionalities.

The evaluator receives recorded user action data and evaluates the received user action data, e.g., it can be evaluated whether or not a sound source has been correctly identified. The evaluation result is represented by evaluation data that are provided for further processing or transmission.

The evaluator is operationally connected to a visual signal output transducer. Via the visual signal output provide a visual output signal representing visual content that complements the directional sound signal can be presented to a user. According to different sound scenes, a complementing visual output signal can be provided to a user as visual input in form of a, e.g., a video. For example, if a nature scene is provided to a user a respective visual output signal may represent a forest with birds singing in the tree. A user of the training arrangement may then localize a bird that sits in a tree and sings. The visual input represented by a visual output signal can be realistic, e.g., recorded with a 360° camera or programmed or comic looking. Preferably, a sound scene is mapped on the visual output signal.

The visual signal output transducer is configured to receive the evaluation data and to provide a visual output signal that is based on the provided evaluation data. The visual output signal is provided to a user of the training arrangement. A visual output signal that is based on the evaluation data may represent a confirmation that a sound source has been correctly identified.

The training arrangement can be used for training a user of the hearing aid system in identifying or locating a sound source, in particular in form of a training game, for fitting the hearing device of the hearing aid system to a user or for training a user of the hearing aid system in using the hearing device in different sound environments. The directional sound signal can also represent a prompt for a user to find a specific sound source, in particular if the training arrangement is used for a training game.

Due to the imitation of realistic scenes, the training arrangement may have the advantage that the immerse experience creates direct memories. By using close to reality scenes, users might memorize more easily how to behave in specific listening situations, e.g., how to position them with respect to a sound source to gain a better hearing experience. As a consequence, the educational impact on a user of the training arrangement may be improved. In particular, this may be of relevance for getting used more quickly to a new hearing device or to make a better use of a hearing device right from the start of use. To improve a training effect, achievement results or other progress markers may be provided to a user, e.g., by the evaluator or by the visual signal output transducer.

According to a second aspect of the invention, the object is achieved by a method for providing a visual output signal via a visual signal output transducer that is operationally connected to a hearing device, wherein the method comprises
- receiving an electric audio input signal, wherein the electric audio input signal represents information about the sound environment of the hearing device,
- processing the electric audio input signal, wherein processing the electric audio input signal includes
- analyzing the electric audio input signal with respect to the information about the sound environment represented by the electric audio input signal,
   - extracting a signal fragment from the analyzed electric audio input signal, wherein the extracted signal fragment represents information about the sound environment,
   - generating a visual signal from the extracted signal fragment, wherein the visual signal depicts information about the sound environment represented by the extracted signal fragment, and
   - providing the visual signal, and
- providing a visual output signal that is based on the visual signal and that can be perceived as visual input by a user.

Optionally, the method of the second aspect further comprises
- a receiving a visual input signal,
   - processing the visual input signal, wherein processing the visual input signal includes modifying the visual input signal in dependence on the visual signal, and
- providing a modified visual input signal to the visual signal output transducer.

In an embodiment, the method further comprises
- monitoring a feature of the hearing aid system or of the received electric audio input signal or both,
- identifying a potential need of assistance for a user of the hearing aid system based on the monitored feature or the received electric audio input signal or both,
- selecting an assistance output signal that represents assistance information about the identified potential need of assistance from a set of assistance output signals, and
- providing a visual output signal that is based on the selected assistance output signal or providing an audio output signal that is based on selected assistance output signal or both.

Optionally, the method further comprises
- identifying a fulfilment of an identified potential need of assistance based on the monitored feature or the received electric audio input signal or both, and
- providing a need fulfilment output signal.

Optionally the method further comprises
- recording monitoring data representing at least one of an identified potential need of assistance, steps performed to satisfy an identified potential need of assistance and a fulfilment of an identified potential need of assistance.

In an embodiment, the method further comprises
- providing and controlling at least one directional sound signal,
   - recording user action data representing a user action, and
- receiving and evaluating the recorded user action data and providing evaluation data representing an evaluation result, and
- providing a visual output signal representing visual content that complements the directional sound signal or providing a visual output signal based on the provided evaluation data or both.

Optionally, the method further comprises
- prompting a user to identify a specific sound source represented by the directional sound signal
- providing a rating signal representing an assessment of how well a specific sound source has been identified.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
Fig. 1a: is a schematic diagram of a preferred hearing aid system according to a first aspect of the invention;
Fig. 1b: is a schematic diagram of a preferred hearing aid system comprising a binaural hearing device;
Fig. 2: is a schematic diagram of a preferred hearing aid system comprising a visual signal input and a visual signal processing unit;
Fig. 3: is a schematic diagram of a preferred hearing aid system comprising a visual signal input and a visual signal processing unit;
Fig. 4: is a schematic diagram of a preferred hearing aid system comprising a monitoring unit;
Fig. 5: is a schematic diagram of a preferred training arrangement;
Fig. 6: is a schematic diagram of the preferred steps to be performed according to the second aspect of the invention in order to provide a visual output signal to a user of a hearing aid system.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

A hearing device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing device is adapted to be worn in any known way. This may include i) arranging a unit of the hearing device behind the ear with a tube leading air-borne acoustic signals or with a receiver/ loudspeaker arranged close to or in the ear canal such as in a Behind-the-Ear type hearing aid or a Receiver-in-the Ear type hearing aid, and/ or ii) arranging the hearing device entirely or partly in the pinna and/ or in the ear canal of the user such as in a In-the-Ear type hearing aid or In-the-Canal/ Completely-in-Canal type hearing aid, or iii) arranging a unit of the hearing device attached to a fixture implanted into the skull bone such as in Bone Anchored Hearing Aid or Cochlear Implant, or iv) arranging a unit of the hearing device as an entirely or partly implanted unit such as in Bone Anchored Hearing Aid or Cochlear Implant.

A hearing device may be part of a "hearing system", which refers to a system comprising one or two hearing devices, disclosed in present description, and a "binaural hearing system" refers to a system comprising two hearing devices where the devices are adapted to cooperatively provide audible signals to both of the user's ears. The hearing system or binaural hearing system may further include auxiliary device(s) that communicates with at least one hearing device, the auxiliary device affecting the operation of the hearing devices and/or benefitting from the functioning of the hearing devices. A wired or wireless communication link between the at least one hearing device and the auxiliary device is established that allows for exchanging information (e.g. control and status signals, possibly audio signals) between the at least one hearing device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing device. The remote control is adapted to control functionality and operation of the at least one hearing devices. The function of the remote control may be implemented in a SmartPhone or other electronic device, the SmartPhone/ electronic device possibly running an application that controls functionality of the at least one hearing device.

In general, a hearing device includes i) an input unit such as a microphone for receiving an acoustic signal from a user's surroundings and providing a corresponding input audio signal, and/or ii) a receiving unit for electronically receiving an input audio signal. The hearing device further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing devices, the output unit may include one or more output electrodes for providing the electric signals such as in a Cochlear Implant.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follows.

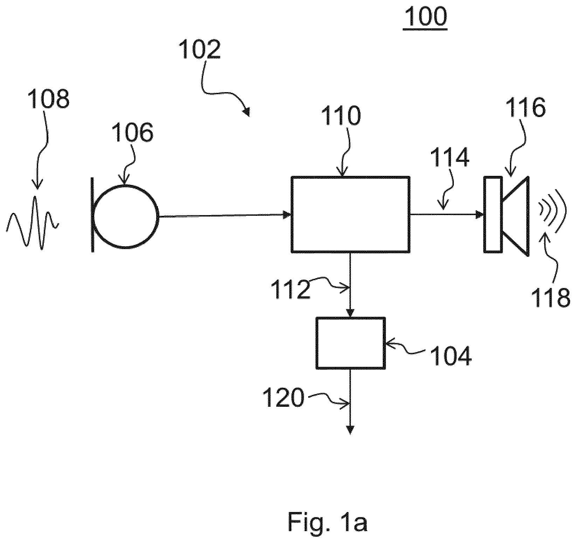

In figure 1a, a schematic diagram of the preferred hearing aid system 100 according to the first aspect of the invention is shown. The hearing aid system 100 comprises the hearing device 102 operationally connected to a visual signal output transducer 104. The hearing device comprises an audio signal input 106 that is configured to receive an electric audio input signal 108.

Preferably, the audio signal input 106 is connected to an input transducer (not shown) that is configured to receive an incoming sound signal and to convert the received sound signal into an electric audio input signal. Thus, the electric audio input signal 108 is a sound-representing electric audio signal. In other words, characteristics of the electric audio input signal 108 represents information about the sound signal received by an input transducer (not shown). The input transducer (not shown) can for example be a microphone that can also be a directional microphone with two microphones or two sound inlets. Instead of being connected to a microphone the audio signal input 106 can also be connected to a signal receiver (not shown) for a wireless reception of electric audio signals, e.g., via Bluetooth.

The hearing device 102 comprises an electric audio input signal processing unit 110 that is operationally connected to the audio signal input 106. The audio input signal processing unit 110 is configured to process the electric audio input signal 108. Processing the electric audio input signal 108 includes analyzing the electric audio input signal 108 with respect to the information about the sound environment represented by the characteristics of the electric audio input signal 108. The electric audio input signal 108 can be analyzed using signal processing techniques such as frequency filtering or noise reduction. An analysis of the electric audio input signal 108 can also comprise executing a pattern recognition algorithm, e.g., for analyzing a speech pattern.

Processing the electric audio input signal 108 also includes extracting a signal fragment from the analyzed electric audio input signal. Characteristics of a signal fragment represent specific information about a sound source of the sound environment. For example, the electric audio input signal 108 representing information about the sound environment may represent specific information about a sound source of the sound environment. A sound source may emit a sound signal with a well-defined starting point and end point, e.g., in time or frequency. The sound signal is part of the sound environment which is represented by the received electric audio input signal 108. A signal fragment, representing the information about a specific sound signal can be extracted from the electric audio input signal 108 during processing of the electric audio input signal 108.

Processing the electric audio input signal 108 also includes generating of visual signal 112 from the extracted signal fragment. The visual signal 112 depicts information about the sound environment, e.g., about a specific sound sauce, represented by the characteristics of the extracted signal fragment. The visual signal can be an analog or digital video signal or it can encode a graphics or image or it can encode text information. The visual signal can also encode a graphical object like a dot, an arrow or a square. The visual signal can also encode a voice command.

Preferably, processing the electric audio input signal 108 also includes executing one or more processing algorithms to obtain a processed electric audio signal, e.g., that can be an electric audio signal that is filtered or amplified in selected frequency bands. After processing, the processed electric audio signal 114 is provided to an audio signal output transducer 116. The audio signal output transducer 116 provides an audio output signal 118 that is based on the processed electric audio signal and that can be perceived as sound by a user.

The audio signal output transducer can be a receiver that emits sound to stimulate a user's eardrum. A respective hearing device can be implemented, e.g., as a behind-the-ear (BTE) hearing device which typically has the microphone arranged behind the ear of a user or as an in-the-ear (ITE) hearing device which typically has the microphone arranged in the ear of a user. In both cases a speaker is typically placed inside a user's ear canal to stimulate the eardrum.

In an embodiment of the shown hearing aid system, the hearing device comprises an implantable part such as in case of a cochlea implant or an auditory brainstem implant. In a cochlea implant, the output transducer can comprise a stimulation pulse generator and an electrode array comprising a number of electrodes for stimulating the cochlea nerve with electric pulses. If the hearing device is an auditory brainstem implant, the output transducer is configured to stimulate the brainstem of a user. In both cases, the audio output signal provided by the output transducer can be perceived as sound by a user.

The operationally connected visual signal output transducer 104 receives the visual signal 112 provided by the electric audio input signal processing unit 110. The visual signal output transducer is configured to provide a visual output signal 120 that is based on the visual signal 112. The visual output signal 120 can be perceived as visual input by a user. The visual output signal 120 can represent visual input in form of text, e.g., text deduced from spoken words represented by the received electric audio input signal 108. The visual output signal 120 can also represent visual input in form of a video, an animation, a graphics or an image. For example, if the characteristics of a signal fragment extracted from the analyzed electric audio input signal is identified with a car noise, a visual output signal representing a car can be provided as visual input to a user. The electric audio input signal 108 can also represent a voice command. A respective electric audio input signal 108 can be analyzed by the audio input signal processing unit 110 and respective signal fragments can be extracted.

The visual signal 112 generated from the extracted signal fragments can encode the execution information of a voice command represented by the characteristics of the received electric audio input signal 108. The visual signal output transducer 104 converts the encoded execution information represented by the visual signal 112 into a respective visual output signal 120 which is then provided to a user as visual input. For example, a voice command can concern the cleaning of the hearing device 102. A respective visual output signal 120 can represent a video showing how to clean the hearing device, wherein the video is presented as visual input to a user.

The visual signal output transducer 104 can be an external electric device that can be either wire-connected or wireless-connected to the hearing device 102 for allowing an exchange of data signals between both devices.

The visual signal output transducer 104 can provide the visual output signal 120 through a display such that the user perceives the visual input through the display. The visual signal output transducer 104 can also be implanted into a user's body, e.g., as a stimulation unit comprising stimulation electrodes for stimulating the user's brain.

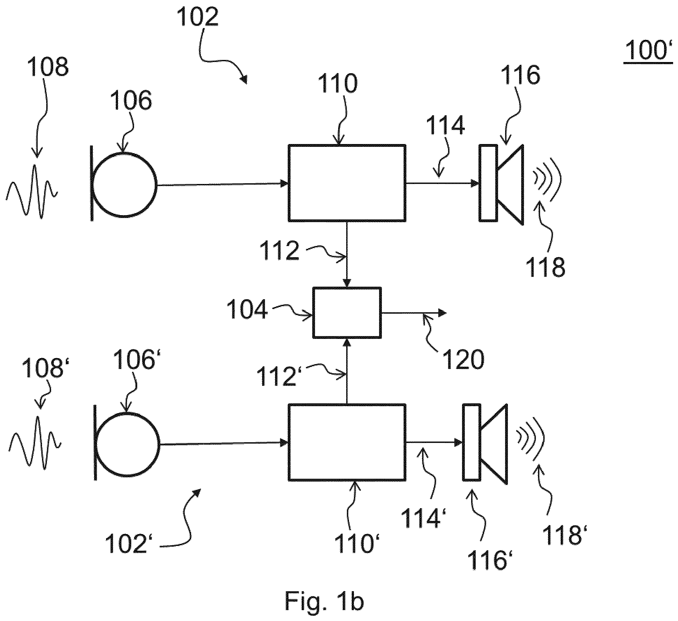

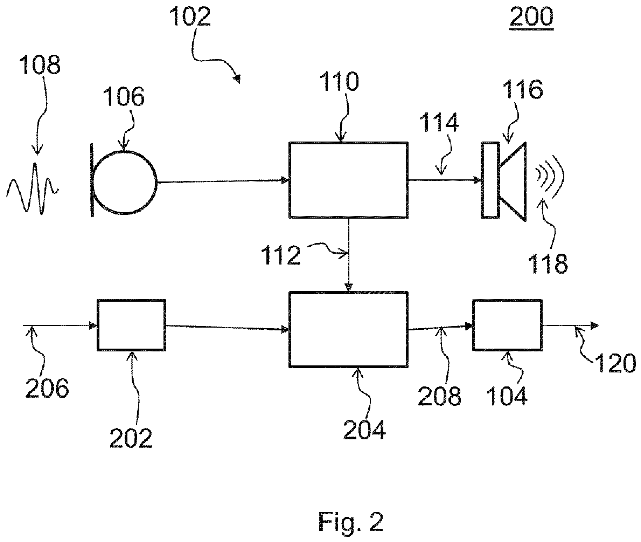

In figure 1b, a schematic diagram of a preferred hearing aid system 100' comprising a binaural hearing device with a first hearing device 102 which is configured as described with respect to figure la, a second hearing device 102' that can be identical to the first hearing device 102 and visual signal output transducer 104 is shown. Both hearing devices 102 and 102' receive respective electric audio input signals 108 and 108'. Both received electric audio input signals 108 and 108' are processed in the respective audio input signal processing units 110 and 110'. Processing the electric audio input signals 108 and 108' includes analysing the electric audio input signals 108 and 108', extracting fragments from the analysed electric audio input signals 108 and 108' and generating respective visual signals 112 and 112' as described with respect to figure 1a. Both visual signals 112 and 112' are fed to the operationally connected visual signal output transducer 104 that provides a visual output signal 120 that is based on the visual signals 112 and 112'. The visual output signal 120 can also be based on only one of the visual signals 112 and 112'.In figure 2, a schematic diagram of a preferred hearing aid system 200 comprising a visual signal input 202 and a visual input signal processing unit 204 is shown. The hearing aid system 200 comprises a hearing device 102 with an audio signal input 106 for receiving an electric audio input signal 108, an electric audio input signal processing unit 110 for processing the electric audio input signal 108 and an audio signal output transducer 116 for providing an audio output signal 118 that is based on the processed electric audio signal 114 provided by the electric audio input signal processing unit 110. As described with respect to the embodiment of a hearing at system shown in figure 1a and figure 1b, processing the electric audio input signal includes analyzing the electric audio input signal 108, extracting a signal fragment from the analyzed electric audio input signal and generating and providing a visual signal 112, wherein the visual signal 112 depicts information about the sound environment represented by the extracted signal fragment.

The visual signal input 202 is configured to receive a visual input signal 206. The visual signal input 202 can be, e.g., a camera, LIDAR (light detection and ranging) or an auxiliary device for receiving a visual input signal. The visual input signal 206 is processed in the visual input signal processing unit 204. The visual input signal processing unit 204 is operationally connected to the electric audio input signal processing unit 110 for receiving the provided visual signal 112.

Processing of the visual input signal 206 in the visual input signal processing unit 204 includes modifying the visual input signal 206 in dependence on the visual signal 112 received from the electric audio input signal processing unit 110 and providing a modified visual input signal 208 to an operationally connected visual signal output transducer 104 which is configured as described with respect to the embodiment of a hearing at system shown in figures 1a and 1b. For example, the visual signal can encode an arrow which indicates the position or direction of an object represented by the visual output signal 120. A user, who perceives a visual input according to the modified visual output signal 208, perceives the visual input according to the visual input signal 206 that has been modified with a respective visual signal 112. In particular, if a visual output signal 104 that is based on a modified visual output signal 208 and a complementary audio output signal provided to a user, the visual input signal 206 can be modified in that the visual output signal 104 is synchronized with respect to the audio output signal 118.

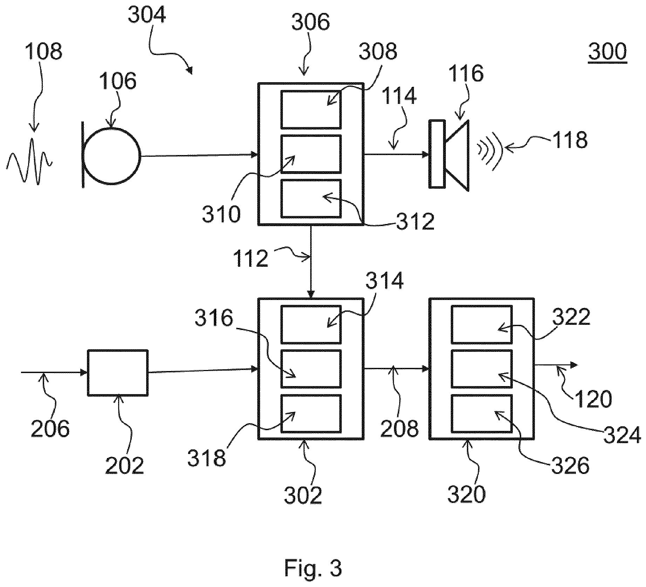

In figure 3, a schematic diagram of a preferred hearing aid system 300 comprising a visual signal input 202 and a visual signal processing unit 302 is shown. The hearing aid system 300 shown, comprises a hearing device 304 with an audio signal input 106 for receiving and electric audio input signal 108 and electric audio input signal processing unit 306 for processing the received electric audio input signal 108 and an audio signal output transducer 116 for providing an audio output signal 118 that this based on the processed electric audio signal 114. The audio signal input 106, the electric audio input signal processing unit 306 and the audio signal output transducer 116 can be configured at as described with respect to the figures la, 1b or 2. In particular, the electric audio input signal processing unit is configured to analyze the electric audio input signal 108, to extract a signal fragment from the analyzed electric audio input signal and to generate and provide a visual signal 112 that depicts information about the sound environment represented by the extracted signal fragment. However, the electric audio input signal processing unit 306 further comprises at least one of an audio input speech identifier 308, an auditory object identifier 310 and an auditory object localizer 312.

The audio input speech identifier 308 is configured to identify speech information represented by a signal fragment extracted from the analyzed electric audio input signal and to transform the signal fragment that represents speech information into a text representing output signal. The speech information represented by a signal fragment can originate from words, e.g., spoken by a user of the hearing at system 300 or by a communication partner.

The auditory object identifier 310 is configured to identify sound information about the sound environment represented by the signal fragment extracted from the analyzed electric audio input signal with an auditory object. The auditory object identifier 310 is further configured to transform the signal fragment that represents the auditory object information into an auditory object representing output signal. An auditory object can be an object that can be identified as a sound source, for example the noise of the car can be identified with the car itself. The singing of a bird can be identified with the bird who sings.

The auditory object localizer 312 is configured to extract position data from the analyzed electric audio input signal. The position data represent a position or directionrelative to the hearing device 304 of an auditory object represented by a respective signal fragment. In other words, the auditory object localizer 312 associates and auditory object with a position or a direction relative to the hearing device 304. The visual signal input 202 comprised in the hearing at system 300 is configured to receive a visual input signal 206. The visual input signal 206 is processed in the visual input signal processing unit 302. As described with respect to figure 2, processing the visual input signal 206 includes modifying the visual input signal 206 independence on the visual signal 112 provided by the electric audio input signal processing unit 306.

The visual input signal processing unit 302 comprises at least one of a visual input signal speech analyzer 314, a visual object analyzer 316 and a visual object localizer 318. The visual input signal speech analyzer 314 is configured to analyze the visual input signal 206 with respect to lip movements and to provide a lip movement representing output signal. The lip movement representing output signal represents information about the lip movement. It is preferred, that the generated lip movement representing output signal is used to perform lip-to-text processing and that the output of the lip-to-text processing is fed into a text-to-speech synthesizer that is configured to generate a speech signal representing the lip movements.

The visual object analyzer 316 is configured to analyze the visual input signal with respect to a visual object represented by the visual input signal 206. Based on a recognized visual object represented by the visual input signal 206, a visual object representing output signal can be generated which can be used for further processing.

The visual object localizer 318 is configured to extract spatial coordinates from the visual input signal 206 that define a position or a direction of a visual object represented by the visual input signal 206 with respect to the position or direction of the hearing device 304. In other words, the visual object localizer 318 is configured to associate a visual object represented by a visual input signal 206 with a position relative to the hearing device 304. As described with respect to figures la, 1b or 2, the hearing aid system 300 comprises a visual signal output transducer 320 that is configured to provide a visual output signal 120 that is based on the visual signal 112. In particular, the visual output signal 120 provided by the visual signal output transducer 320 is based on a visual input signal 206 that is modified in dependence on the visual signal 112, thus, constituting the modified visual input signal 208.

The visual signal output transducer 320 comprises at least one of an artificial object positioner 322, a lip movement modifier 324 and an object placement informer 326. In general, the artificial object positioner 322, the lip-movement modifier 324 and the object placement informer 326 are configured to modify a visual input signal 206 that represents an unaltered visual input with a visual signal 112 that depicts information about the sound environment represented by an extracted signal fragment. Hence, the modified visual output signal 208, in general, is a superposition of a real image or image sequence with a virtual image or image sequence. In particular, as the visual signal 112 used for modifying is based on the information represented by a characteristics of a signal fragment extracted from the electric audio input signal 108, the visual input signal 206 is modified with respect to the information represented by the signal fragment extracted from the electric audio input signal 108.

The artificial object positioner 322 modifies a visual input signal 206 by means of a visual signal 112 which represents information on, e.g., where to position an artificial object, like a dot, a square, or an arrow, in the visual output signal. A respective visual signal 112 can be an object representing signal provided by an auditory object identifier 308 or position data representing a position or direction relative to the hearing device of an identified auditory object provided by an auditory object localizer 213. Based on an object representing signal and respective position data, the visual input signal 206 can be modified to signal to a user via, e.g., an arrow, that a certain type of auditory object has been identified at a certain position or direction relative to the hearing device 304. It might be useful, to implement a colour coding scheme that relates a certain type of auditory object with a respective colour. For example, if a musical object is identified as well as its location, a dot may indicate the direction and a certain colour of the dot may indicate that it is a musical object. A possible colour-coding scheme may be implemented as blue for music, green for birds, red for cars or other motor vehicles, purple for voices and grey for noise.

The lip-movement modifier 324 modifies the lip movements represented by a visual input signal 206 in dependence on a visual signal 112 which represents speech information. In particular, the lip movements represented in a characteristics of a visual input signal 206 can be modified by a text representing signal provided by an audio input speech identifier 308. The text representing signal can be used to enhance the lip movements represented by the characteristics of a visual input signal 206 and to provide a modified visual output signal 208. Thereby, visual speech cues can be more salient in the modified output signal 208 that is provided as visual output signal 120 to a user. It is preferred, that the lip movements represented by the characteristics of a visual input signal 206 are modified in real-time. It is of particular advantage, if the lip movements are modified synchronous to a possible speech delay such that a user does not perceive a delay between the perceived audio output signal 118 and the perceived visual output signal 120.

The object placement informer 326 modifies the visual input signal 206 with an object placement informer signal that represents information about a placement of an object. Via the audio signal input, an electric audio input signal 108 representing sound information of an auditory object is received. An auditory object identifier 310 provides a respective auditory object representing signal and an auditory object localizer 312 may provide position data representing a position or direction of a respective auditory object relative to the hearing device. Based on an auditory object representing signal and respective position data, an object placement informer signal may modify a visual input signal 206 in that a user is alerted about an identified object, e.g., by an arrow included in the visual output signal 120 pointing in the direction of the identified object.

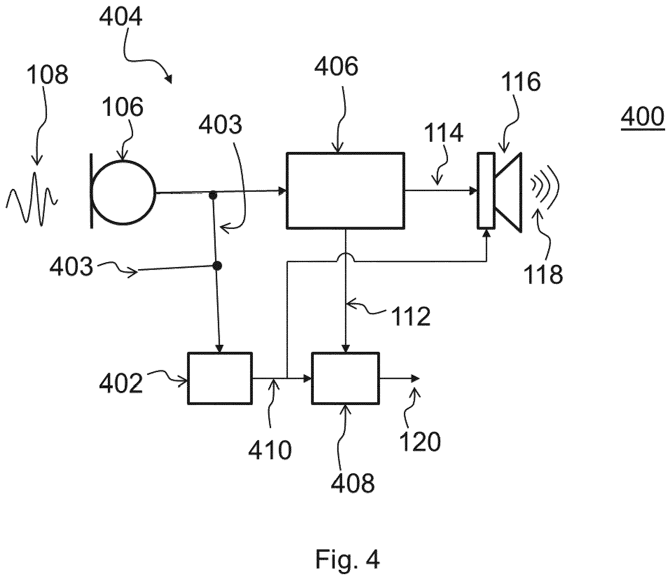

In figure 4, a schematic diagram of the preferred hearing aid system 400 comprising a monitoring unit 402 is shown. The hearing aid system 400 comprises a hearing device 404 with an audio signal input 106 for receiving an electric audio input signal 108, and an electric audio input signal processing unit 406 for processing the received electric audio input signal 108 and an audio signal output transducer 116 for providing an audio output signal 118 that this based on a processed electric audio signal 114 provided by the electric audio input signal processing unit 406. In particular, the electric audio input signal processing unit 406 can be configured according to an electric audio input signal processing unit as described with respect to figures 1a, 1b, 2, or 3.

The electric audio input signal processing unit 406 is at least configured to analyze the electric audio input signal 108, to extract a signal fragment from the electric audio input signal and to generate and provide a visual signal 112 that depicts information about the sound environment as represented by an extracted signal fragment.

The hearing at system 400 may further comprise a visual signal input (not shown) for receiving a visual input signal and a visual input signal processing unit (not shown) as described with respect to figure 2 or 3. The hearing aid system 400 comprises a monitoring unit 402 that is configured to monitor a feature 403 of the hearing at system 400 or of the received electric audio input signal 108 or both.

The monitoring unit 402 is further configured to identify a potential need of assistance for the user of the hearing aid system 400 based on a monitored feature 403 of the electric audio input signal or both. The monitoring unit 402 is further configured to select an assistance output signal 410 that represents assistance information related to the identified potential need of assistance from a set of assistance output signals. The monitoring unit 402 is operationally connected to the visual signal output transducer 408 and to the audio signal output transducer 116.

The visual signal output transducer 408 can be configured as the visual signal output transducer described in figure 3. The visual signal output transducer 408 is configured to provide a visual output signal 120 that is based on the assistance output signal 410. The audio signal output transducer 116 is configured to provide an audio output signal that this based on the selected assistance output signal 410.

The monitoring unit 402 is configured to provide assistance information about when and how to maintain the hearing device 404. In particular, the monitoring unit 402 provides a selected assistance output signal 410 representing information on, e.g., how to solve or carry out a specific task to satisfy a potential need of assistance of a user. A potential need of assistance can be identified either from monitoring the feature of the hearing device 404 or by receiving a voice prompt represented by a received electric audio input signal 108. Assistance can be provided audibly in form of the audio output signal 118 that this based on a respective selected assistance output signal 410. Assistance can also be provided visually, e.g., through a visual signal output transducer that, e.g., comprises a display for presenting visual input represented by a visual output signal 120 that is based on a selected assistance output signal 410. A respective visual signal output transducer 408 can be a Smartphone or a virtual reality headset operationally connected to the hearing device 404.

In particular, a step-by-step guidance can be provided on a display, presenting tasks to be performed to satisfy and identify potential need of assistance. It is also possible to provide assistance visually and audibly to a user, e.g., in form of a video complemented with spoken explanations.

Features of the hearing device 404 that may be monitored by the monitoring unit 402 comprise the signal quality of the received signal or of the output signal, e.g., concerning the signal-to-noise ratio, electronic failure, software failure, the battery status, the fitting of the hearing device, the currently applied hearing device program or receiver occlusion. From the listed examples of monitored features, a potential need of assistance may be deduced. A potential need of assistance may also be identified by monitoring the received electric audio input signal 108. If a user or a communication partner asks for support, a potential need of assistance may be identified via voice prompt recognition. If for example, a user asks for help for, e.g., changing the battery, a respective assistance output signal can be selected according to the identified need.

The monitoring unit may comprise or access a memory (not shown) comprising a predefined set of assistance output signals from which a respective assistance output signal can be selected according to an identified need of assistance.

A visual output signal 120 that is based on the selected assistance output signal 410 can be presented in form of a video or a graphic that explains how to solve or carry out a specific task to satisfy the potential need of assistance. An audio output signal 118 that is based on the selected assistance output signal 410 can be presented in form of a spoken explanation of how to solve a specific task to satisfy the potential need of assistance.

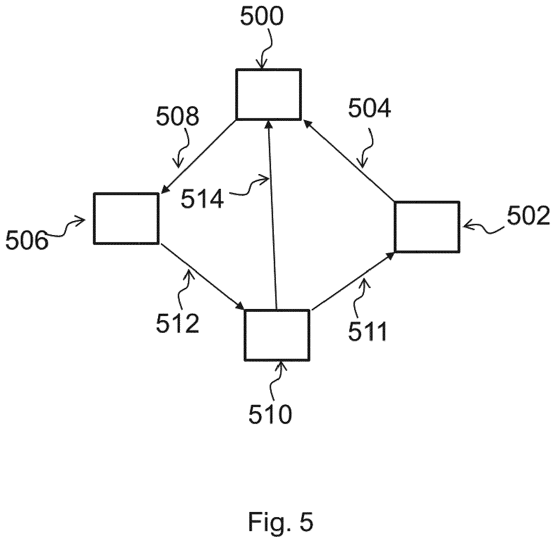

In figure 5, a schematic diagram of a preferred training arrangement 550 is shown. The training arrangement 550 comprises a hearing aid system 500 that can be configured according to a hearing aid system as described with respect to figures 1a, 1b, 2, 3, and 4. The training arrangement 550 further comprises a sound source 502 for providing at least one directional sound signal 504. The training arrangement 550 further comprises a user interface 506 that is configured to record user action data 508 representing a user action. The training arrangement 550 further comprises an evaluator 510 that is operationally connected to the sound source 502, to control 511 the directional sound signal 504 provided by the sound source 502, the user interface 506, to receive and evaluate the recorded user action data 512 and to provide evaluation data 514 representing an evaluation result, and to a visual signal output transducer (not shown) of the hearing aid system 500. Via the visual signal output transducer (not shown), a visual output signal representing visual content that complements the directional sound signal 504 or a visual output signal based on the provided evaluation data 514 or both can be provided to a user of the hearing aid system 500.

The sound source 502 can be surround sound headphones or a speaker or speaker arrangement for providing a directional sound signal 502 that can be perceived by a user of the training arrangement 550.

The user interface 506 can comprise or can be connected to an eye movement tracker. Via eye tracking, the point of gaze or the motion of an eye relative to the head can be measured and analyzed. For example, if a visual signal output transducer is implemented within a virtual reality headset comprising or connected to an eye movement tracker, it can be tracked whether or not a user looks at an object that emits a sound signal. Thus, via an eye movement tracker it can be verified that a user found a correct sound source.

The user interface 506 can comprise or can be connected to a controller. By pressing a button of the controller a correct sound source or object identification can be registered. For example, if a user looks at an identified sound source as detected by an eye movement tracker and presses a button of a controller it can be verified that a user found the correct sound source.

The training arrangement 550 can be used to train localizing or identifying sound sources. The training arrangement can also be used for users to learn how to position themselves with respect to different sound environment situations. The training arrangement 550 can also be used for training communications strategies with respect to different communication partners or sound environments. The training arrangement can also be used for hearing rehabilitation training.

The evaluator 510 can be a home video game console. The evaluator 510 can also be a personal computer or an alternative suitable electronic device. Via the evaluator 510 the sound source 502 can be controlled, e.g., the directionality of a sound signal 504. In particular, different sound scenes such as a nature scene, a city scene, a home scene, and a restaurant scene can be provided to the sound source and a respective sound signal can be emitted. A sound scene can be rather complex, e.g., comprising simultaneous sound sources and directionalities.

The evaluator 510 receives recorded user action data 512 and evaluates the received user action data, e.g., it can be evaluated whether or not a sound source has been correctly identified. The evaluation result is represented by evaluation data 514 that are provided for further processing or transmission, e.g., to a visual signal output transducer (not shown).

The training arrangement 550 can be used for training a user of the hearing aid system in identifying or locating a sound source, in particular in form of a training game, for fitting a hearing device of the hearing aid system 500 to a user or for training a user of the hearing aid system 500 in using a hearing device in different sound environments. The directional sound signal 504 can also represent a prompt for a user to find a specific sound source, in particular if the training arrangement is used for a training game.

Due to the imitation of realistic scenes, the training arrangement 550 may have the advantage that the immerse experience creates direct memories. By using close to reality scenes, users might memorize more easily how to behave in specific listening situations, e.g., how to position them with respect to a sound source to gain a better hearing experience. As a consequence, the educational impact on a user of the training arrangement 550 may be improved. In particular, this may be of relevance for getting used more quickly to a new hearing device or to make a better use of a hearing device right from the start of use. To improve a training effect, achievement results or other progress markers may be provided to a user, e.g., by the evaluator 510 or by a visual signal output transducer (not shown).

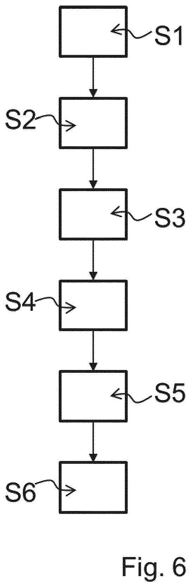

In figure 6, a schematic diagram of the preferred steps to be performed according to a second aspect of the invention in order to provide a visual output signal to a user of a hearing aid system is shown.

In a first step S1 an electric audio input signal is received. The electric audio input signal represents information about the sound environment of the hearing device. In a subsequent step the electric audio input signal is processed. The processing step includes analyzing S2 the electric audio input signal with respect to the information about the sound environment represented by the electric audio input signal, extracting S3 a signal fragment from the analyzed electric audio input signal, wherein the extracted signal fragment represents information about the sound environment, generating S4 a visual signal from the extracted signal fragment, wherein the visual signal depicts information about the sound environment represented by the extracted signal fragment, and providing S5 the visual signal. In a further step S6, a visual output signal is provided. The visual output signal is based on the visual signal and can be perceived as visual input by a user.

Optionally, the method may further comprise the steps of receiving a visual input signal, processing the visual input signal, wherein processing the visual input signal includes modifying the visual input signal in dependence on the visual signal, and providing a modified visual input signal to a visual signal output transducer.

Alternatively or additionally to the steps of receiving and processing a visual input signal and providing a modified visual input signal, the method may comprise the steps of monitoring a feature of the hearing aid system or of the received electric audio input signal or both, identifying a potential need of assistance for a user of the hearing aid system based on the monitored feature or the received electric audio input signal or both, selecting an assistance output signal that represents assistance information about the identified potential need of assistance from a set of assistance output signals, and providing a visual output signal that is based on the selected assistance output signal or providing an audio output signal that is based on selected assistance output signal or both.

Optionally, the method may comprise the steps of identifying a fulfilment of an identified potential need of assistance based on the monitored feature or the received electric audio input signal or both, and providing a need fulfilment output signal.

Optionally the method may comprise the steps of recording monitoring data representing at least one of an identified potential need of assistance, steps performed to satisfy an identified potential need of assistance and a fulfilment of an identified potential need of assistance.

## Claims

1. A hearing aid system (100) comprising:
a hearing device with
- an audio signal input, for receiving an electric audio input signal, wherein the electric audio input signal has characteristics that depend on a sound environment and therefore represents information about the sound environment of the hearing device,
- an audio input signal processing unit operationally connected to the audio signal input and being configured for processing the electric audio input signal, said processing including
- analyzing the electric audio input signal with respect to the information about the sound environment represented by the characteristics of the electric audio input signal,
- extracting a signal fragment from the analyzed electric audio input signal, wherein characteristics of the extracted signal fragment represents information about the sound environment,
- generating a visual signal from the extracted signal fragment, wherein the visual signal depicts information about the sound environment represented by the extracted signal fragment, and
- providing a processed electric audio signal or the visual signal or both,
- an audio signal output transducer operationally connected to the audio input signal processing unit, for providing an audio output signal that is based on the processed electric audio input signal and that can be perceived as sound by a user,
and
- a visual signal output transducer operationally connected to the audio input signal processing unit, for providing a visual output signal that is based on the visual signal and that can be perceived as visual input by a user.

2. The hearing aid system of claim 1, further comprising
- a visual signal input, for receiving a visual input signal, and
- a visual input signal processing unit operationally connected to the visual signal input and to the electric audio input signal processing unit and to the visual signal output transducer, for
- processing the visual input signal, wherein processing the visual input signal includes modifying the visual input signal in dependence on the visual signal provided by the electric audio input signal processing unit, and
- providing a modified visual input signal to the visual signal output transducer.

3. The hearing aid system of claim 1 or 2, wherein the audio input signal processing unit comprises at least of an
- audio input speech identifier, for identifying speech information represented by a signal fragment extracted from the analyzed electric audio input signal and for transforming the signal fragment that represents speech information into a text representing output signal,
- auditory object identifier, for identifying the sound information about the sound environment represented by a signal fragment extracted from the analyzed electric audio input signal with an auditory object and transforming the signal fragment that represents the auditory object information into an auditory object representing signal, and
- auditory object localizer, for extracting position data from the analyzed electric audio input signal, wherein the position data represent a position relative to the hearing device of an auditory object represented by a respective signal fragment.

4. The hearing aid system of claim 2, wherein the visual input signal processing unit comprises at least one of a
- visual input signal speech analyzer, for analyzing the visual input signal with respect to lip movements and for providing a lip movement representing output signal,
- visual object analyzer, for analyzing the visual input signal with respect to a visual object and for providing a visual object representing output signal, and
- visual object localizer, for extracting spatial coordinates from the visual input signal that define a position of a visual object represented by the visual input signal with respect to the position of the hearing device.

5. The hearing aid system of at least one of the preceding claims, wherein the visual signal output transducer comprises at least one of
- an artificial object positioner, that is configured to modify a visual input signal with a respective visual signal which represents information on positioning an artificial object in the visual output signal and to provide a modified visual output signal,
- a lip-movement modifier, that is configured to modify the lip movements represented in a visual input signal in dependence on a visual signal which represents speech information and to provide a modified visual output signal, and
- an object placement informer, that is configured to modify the visual input signal with an object placement informer signal that represents information about a placement of an object and to provide a modified visual output signal.

6. The hearing aid system of at least one of the preceding claims, wherein the hearing aid system further comprises a monitoring unit operationally connected to the visual signal output transducer or operationally connected to the audio signal output transducer or both, wherein the monitoring unit is configured to
- monitor a feature of the hearing aid system or of the received electric audio input signal or both,
- identify a potential need of assistance for a user of the hearing aid system based on the monitored feature of the hearing aid system or of the electric audio input signal or both,
- select an assistance output signal that represents assistance information about the identified potential need of assistance from a set of assistance output signals and provide the selected assistance output signal, wherein
the visual signal output transducer is configured to provide a visual output signal that is based on the selected assistance output signal or the audio signal output transducer is configured to provide an audio output signal that is based on the selected assistance output signal or both.

7. The hearing aid system of claim 6, wherein the monitor unit is configured to identify a fulfilment of an identified potential need of assistance based on the monitored feature or the received electric audio input signal or both and to provide a need fulfilment output signal.

8. The hearing aid system of claim 6 or 7, wherein the monitoring unit is configured to record monitoring data representing at least one of an identified potential need of assistance, steps performed to satisfy an identified potential need of assistance and a fulfilment of an identified potential need of assistance.

9. A training arrangement comprising
- a hearing aid system of at least one of the preceding claims,
- a sound source for providing at least one directional sound signal,
- a user interface that is configured to record user action data representing a user action, and
- an evaluator that is operationally connected to at least one of the
- sound source, to control the directional sound signal provided by the sound source,
- user interface, to receive and evaluate the recorded user action data and to provide evaluation data representing an evaluation result, and
- visual signal output transducer, that is configured to provide a visual output signal representing visual content that complements the directional sound signal or that is configured to provide a visual output signal based on the provided evaluation data or both to a user of the hearing aid system.

10. A use of the training arrangement of claim 11, for training a user of the hearing aid system in identifying or locating a sound source, for fitting the hearing device of the hearing aid system to a user, for training a user of the hearing aid system in using the hearing device in different sound environments.

11. A method for providing a visual output signal via a visual signal output transducer that is operationally connected to a hearing device, wherein the method comprises
- receiving an electric audio input signal, wherein the electric audio input signal represents information about the sound environment of the hearing device,
- processing the electric audio input signal, wherein processing the electric audio input signal includes
- analyzing the electric audio input signal with respect to the information about the sound environment represented by the electric audio input signal,
- extracting a signal fragment from the analyzed electric audio input signal, wherein the extracted signal fragment represents information about the sound environment,
- generating a visual signal from the extracted signal fragment, wherein the visual signal depicts information about the sound environment represented by the extracted signal fragment, and
- providing the visual signal, and
- providing a visual output signal that is based on the visual signal and that can be perceived as visual input by a user.

12. The method of claim 11, further comprising
- a receiving a visual input signal,
- processing the visual input signal, wherein processing the visual input signal includes modifying the visual input signal in dependence on the visual signal, and
- providing a modified visual input signal to the visual signal output transducer.

13. The method of claim 11 or 12, further comprising
- monitoring a feature of the hearing aid system or of the received electric audio input signal or both,
- identifying a potential need of assistance for a user of the hearing aid system based on the monitored feature or the received electric audio input signal or both,
- selecting an assistance output signal that represents assistance information about the identified potential need of assistance from a set of assistance output signals, and
- providing a visual output signal that is based on the selected assistance output signal or providing an audio output signal that is based on selected assistance output signal or both.

14. The method of claim 13, further comprising
- identifying a fulfilment of an identified potential need of assistance based on the monitored feature or the received electric audio input signal or both, and
- providing a need fulfilment output signal.

15. The method of claim 13 or 14, further comprising
- recording monitoring data representing at least one of an identified potential need of assistance, steps performed to satisfy an identified potential need of assistance and a fulfilment of an identified potential need of assistance.

16. The method of at least one of claims 11 to 15, further comprising
- providing and controlling at least one directional sound signal,
- recording user action data representing a user action, and
- receiving and evaluating the recorded user action data and providing evaluation data representing an evaluation result, and
- providing a visual output signal representing visual content that complements the directional sound signal or providing a visual output signal based on the provided evaluation data or both.

17. The method of claim 16, further comprising
- prompting a user to identify a specific sound source represented by the directional sound signal
- providing a rating signal representing an assessment of how well a specific sound source has been identified.
